# EUROPEAN PATENT SPECIFICATION

(11) **EP 2 309 957 B1**
(45) Date of publication and mention of the grant of the patent: **16.04.2014**
(21) Application number: 09785424.4
(22) Date of filing: 31.07.2009
(51) Int. Cl.: A61F 13/00, A61F 13/12

(54) **TRACHEOSTOMY DRESSING**
TRACHEOSTOMIE-VERBAND
PANSEMENT DE TRACHÉOSTOMIE

(30) Priority: 01.08.2008 GB 0814102; 07.05.2009 US 176239 P
(43) Date of publication of application: 20.04.2011
(73) Proprietor: Cambridge University Hospitals NHS Foundation Trust, Cambridge Cambridgeshire CB2 0QQ (GB)
(72) Inventor: CAMERON, Malcolm, Cambridge Cambridgeshire CB21 4RP (GB); CASCARINI, Luke, Beckenham Kent BR3 1LL (GB)
(74) Representative: Miller, David James
(86) International application number: PCT/GB2009/050950
(87) International publication number: WO 2010/013057

(56) References cited:
- WO-A1-90/06735
- WO-A2-2007/092289
- GB-A- 2 413 767
- US-A- 3 464 410
- US-A- 3 811 436
- US-A- 4 463 757
- US-B1- 6 779 525

## Description

This invention relates to a tracheostomy dressing which enables patients to easily locate a healing tracheostomy wound on the neck following decanulation (removal of a tracheostomy tube).

Temporary tracheostomies are used in many areas of surgery and anaesthesia. They are removed (that is, the patient is decanulated) once their usefulness has passed. Following decanulation, it is desirable for the tracheostomy wound to heal as quickly as possible as delay in healing can be problematic for a patient. Delay may be caused by persistent passage of expired air via the tracheostomy wound rather than the larynx (voicebox). If this occurs, the healing wound is disrupted, literally blown open, necessarily slowing down the healing process.

Traditionally, patients have been instructed to apply firm pressure with finger tips or knuckles over the tracheostomy wound to prevent the unwanted passage of air via the healing wound. Even with careful guidance, many patients find it very difficult to locate the wound easily, in particular because the patient cannot see the wound, thus reducing the effectiveness of this action. Dressings are placed over the healing wound which preclude exact location of the wound below by the patient. Such dressings may be bulky or the patients' manual dexterity or sensation may be impaired. In addition, the dressings often cause excoriation of the skin. Consequently, although the patient may attempt to comply with instructions, if pressure is not applied in the correct position the action is futile. If pressure is incorrectly applied by the patient, the dressing can be dislodged with the resultant passage of air and results in exudates passing through the wound. This situation is clearly disadvantageous and significantly impairs the healing process and increases the risk of infection.

GB 2413767 (Queen Victoria Hospital NHS Foundation Trust) discloses a toggle device for use in combination with existing dressings enabling a patient to locate and apply pressure to a healing tracheostomy wound.

The invention relates to a tracheostomy dressing which comprises an integral wound locating device for locating and applying pressure to a healing tracheostomy wound. More particularly, the present invention provides a tracheostomy dressing which comprises an integral wound locating device having a textured portion that is (i) recessed or concave, and/or (ii) ribbed, wherein the integral wound locating device is adapted, in use, to allow for the location of a healing tracheostomy wound and the application of pressure thereto. The present invention and embodiments thereof are set out in the appended claims.

The invention provides a dressing which can be simply applied to a patient without the need to combine a separate wound locating device with wound dressings. Such an integrated dressing provides a significant number of advantages primarily relating to patient comfort and optimised healing. For example, the integrated dressing effectively prevents air and exudate from escaping through the tracheostomy wound during high-pressure expiration such as talking, coughing or sneezing (i.e. the integrated device provides a more effective seal or vacuum when pressure is applied). This will result in greater patient comfort because current tracheostomy patients experience pain and discomfort when air/exudate passes through the wound site. The integrated device provides a product which is less likely to cause ingress of pathogens and therefore reduces the likelihood of infection. The integrated device also results in faster speech restoration because air is correctly directed through the larynx/voicebox rather than through the wound site. Furthermore, the dressing of the invention is more easily and quickly applied to a patient by nursing staff which not only enhances the healing process but also reduces the amount of valuable time used by the nursing staff in this procedure.

References herein to "wound locating device" shall be interpreted as meaning a device for locating and applying pressure to a healing tracheostomy wound.

References herein to "dressing" include all 'medical products which are intended to be applied to a wound and aid in the healing of said wound and prevent further harm or damage. Typically, the dressing should be sterile, breathable, and encourage a moist healing environment.

Examples of suitable dressings include bandages and plasters. Particular dressings which may be suitable for use with the integral wound locating device are described in www.dressings.org and include Acticoat, Actisorb Silver 220, Algisite M, Allevyn, Aquacel, Bactigras, Biatain, Bioclusive, Biofilm, Blenderm, Blue line webbing, Bordered Granuflex, Calaband, Carbonet, Cavi-care, Cellacast Xtra, Cellamin, Cellona, Chlorhexitulle, Cica-Care, Cliniflex, Clinisorb, Coban, Coltapaste, Comfeel, Contreet Non Adhesive, Crevic, Cutinova Hydro, Debrisan, Delta-Cast, Delta-Lite S, Duoderm extra thin, Duoderm signal, Durapore, Elastocrepe, Elset, Flamazine, Fucidin Intertulle, Geliperm, Granuflex (Improved formulation), Granuflex extra thin, Granugel, Gypsona, Hypafix, Icthaband, Icthopaste, Inadine, Intrasite Gel, Iodoflex, Iodosorb, Jelonet, K-Band, K-Lite, K-Plus, Kaltocarb, Kaltostat, LarvE, Lestreflex, Lyofoam, Mefix, Melolin, Mepiform, Mepilex, Mepitac, Mepitel, Mepore, Mesorb, Mesitran, Metrotop, Microfoam, Micropore, Opsite Flexigrid, Opsite IV 3000, Orthoflex, Oxyzyme, Paratulle, Polymem, Profore, ProGuide, Promogran, Quinaband, Release, Scotchcast Plus, Scotchcast Softcast, Serotulle, Setopress, Silastic foam, Silicone N-A, Sofra-Tulle, Sorbsan, Spenco 2nd Skin, Spyroflex, Spyrosorb, Tarband, Tegaderm, Tegagel, Tegapore, Tegasorb, Telfa, Tensopress, Tielle, Transpore, Unitulle, Veinoplast, Veinopress, Versiva, Vigilon, Viscopaste PB7, Xelma and Zincaband.

In one embodiment, the dressing comprises a gauze, a film, a gel, a foam, a hydrocolloid, an alginate, a hydrogel or a polysaccharide paste, granule or bead. In a further embodiment, the dressing comprises a hydrocolloid material. In a yet further embodiment, the dressing comprises a Granuflex dressing (e.g. Bordered Granuflex, Granuflex (Improved formulation) or G ranuflex extra thin) or Duoderm dressing (e.g. Duoderm signal or Duoderm extra thin). Such dressings provide the advantage of adhering to the patient's skin but without causing abrasion or skin excoriation upon removal of the dressing. Additionally, the provision of a hydrocolloid based dressing provides an advantageous vacuum over the tracheostomy wound. Such a vacuum prevents the flow of air and/or exudate through the wound during high-pressure expiration such as talking, coughing or sneezing.

In one embodiment, the dressing may be impregnated with an agent designed to enhance sterility and/or healing. In a further embodiment, the dressing may be impregnated with one or more antiseptic chemicals.

It will be appreciated that references to "integral" include the sale of the dressing having the wound locating device attached rather than the wound locating device constituting a separate item of commerce. Therefore, in one embodiment, the dressing is constructed from a single piece together with the integral wound locating device. In an alternative embodiment, the wound locating device may be constructed separately from the dressing and bonded (e.g. via adhesive or otherwise) to the tracheostomy dressing. In such an embodiment, the wound locating device may be constructed by many methods such as injection moulding or lathe cutting. In such an embodiment the wound locating device may be constructed from a single piece. Constructing the wound locating device from a single piece is advantageous because it simplifies the manufacturing process, reduces cost and may make the wound locating device more hygienic.

In one embodiment, the integral wound locating device is as described in GB 2413767. For example, the wound locating device consists of a shaped contact plate (e.g. having a substantially square outline and a curved cross section) onto which a short stalk is attached anteriorly, wherein the top of the stalk comprises a finger button.

In an alternative embodiment, the integral wound locating device is substantially square, rectangular or circular in shape. In a yet further embodiment, the integral wound locating device is substantially circular in shape. In a further embodiment, the wound locating device is approximately 0.5 to 4cm in diameter. It will be appreciated that smaller sized devices would be envisaged for children (e.g. approximately 0.5 to 1 cm in diameter).

The integral wound locating device additionally comprises a textured portion that is recessed or concave, and/or ribbed. It will be appreciated that the textured portion would have a surface feel which is distinct from the surrounding area.

This embodiment, while being contrary to the protruding toggle described in GB 2413767, provides a number of advantages. For example, the textured portion of the wound locating device can be simply found by moving the tip of a finger over the dressing. Furthermore, the textured portion is more subtle than the toggle described in GB 2413767 which enhances patient compliance and acceptance. The toggle embodiment could easily be knocked and cause mis-alignment of the dressing with respect to the tracheostomy wound. By contrast, the dressing of the invention is less likely to be dislodged because it is substantially flat. Additionally, the textured portion provides a configuration which permits simple moulding from a single piece and is therefore suitable for integral construction within a dressing.

In a further embodiment, the textured portion is a recessed or concave portion. Such an embodiment is advantageous because it makes the wound locating device easier to find due to the shape of the textured portion, and, once found, the tip of a patient's finger is less likely to slip off the wound locating device because the finger tip is located by the recessed or concave shape of the textured portion. Furthermore, a recessed or concave portion is even less likely to snag on for instance clothes and jewellery than the toggle described in GB 2413767 because it does not protrude significantly from the dressing and does not provide an upstanding feature to catch clothing or the like.

In a further embodiment, the textured portion is a ribbed portion. Such an embodiment is also advantageous because it makes the wound locating device easier to find due to the shape of the textured portion, and, once found, the tip of a patient's finger is less likely to slip off the wound locating device because of the resistance created by the ribbed portion. Furthermore, a ribbed portion is less likely to snag on for instance clothes and jewellery than the toggle described in GB 2413767 because it does not protrude from the dressing to the extent that the toggle does.

In a further embodiment, the textured portion is both recessed or concave and ribbed.

It will be appreciated that the textured portion may be any geometric shape suitable to allow rapid sensory knowledge of the exact location of the wound locating device. In a further embodiment, the textured portion is substantially square, rectangular or circular in shape. In a yet further embodiment, the textured portion is substantially circular in shape.

The wound locating device may be constructed from many materials. It should be firm but not necessarily rigid, indeed a slightly flexible material may be preferable for patient comfort and improved function. A slightly flexible material would conform better to the patients' surface anatomy and reduce the risk of air escaping from the edges of the wound locating device. In particular, it has been observed that many patients often present with severe malnourishment which results in fairly deep recesses within their neck regions. In one embodiment, the wound locating device is constructed from a flexible material. In a further embodiment, the wound locating device is constructed from a polyurethane, polyethylene, thermoplastic elastomeric (TPE) material, thermoplastic rubber (TPR) material, a silicone material or a styrene ethylene butylene styrene (SEBS) material.

In one embodiment, the wound locating device is transparent. This embodiment provides the advantage of being able to accurately position the dressing over the tracheostomy wound.

It will be appreciated that optional features applicable to one aspect of the invention can be used in any combination, and in any number. Moreover, they can also be used with any of the other aspects of the invention in any combination and in any number. This includes, but is not limited to, the dependent claims from any claim being used as dependent claims for any other claim in the claims of this application.

The invention will now be described, by way of example only, with reference to the accompanying drawings, in which:
**Figure 1** shows an exploded view of the components of the tracheostomy dressing according to one embodiment of the invention;
**Figure 2** shows a perspective view of the tracheostomy dressing according to one embodiment of the invention;
**Figure 3** shows a side view of the tracheostomy dressing according to one embodiment of the invention; and
**Figure 4** shows a photograph demonstrating an *in situ* position of the tracheostomy dressing according to one embodiment of the invention.

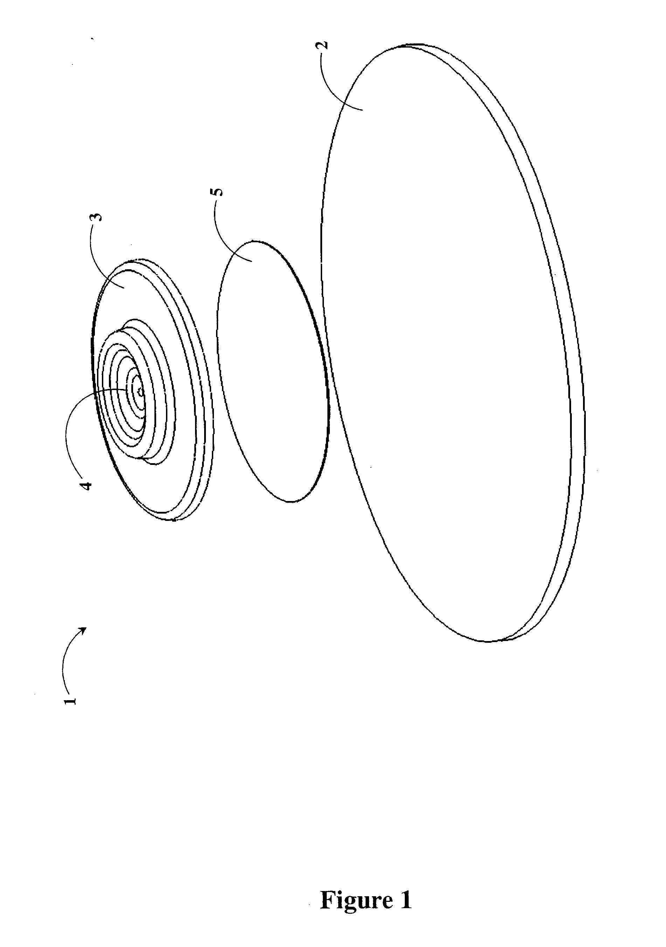

Referring first to Figure 1, a tracheostomy dressing shown generally as 1, comprises a dressing portion 2, a wound locating portion 3 and an adhesive layer 5 for attachment of the wound locating portion 3 to the dressing portion 2. In the embodiment shown in Figure 1, the wound locating portion 3 comprises a series of circular ribs which create a recessed or concave portion 4.

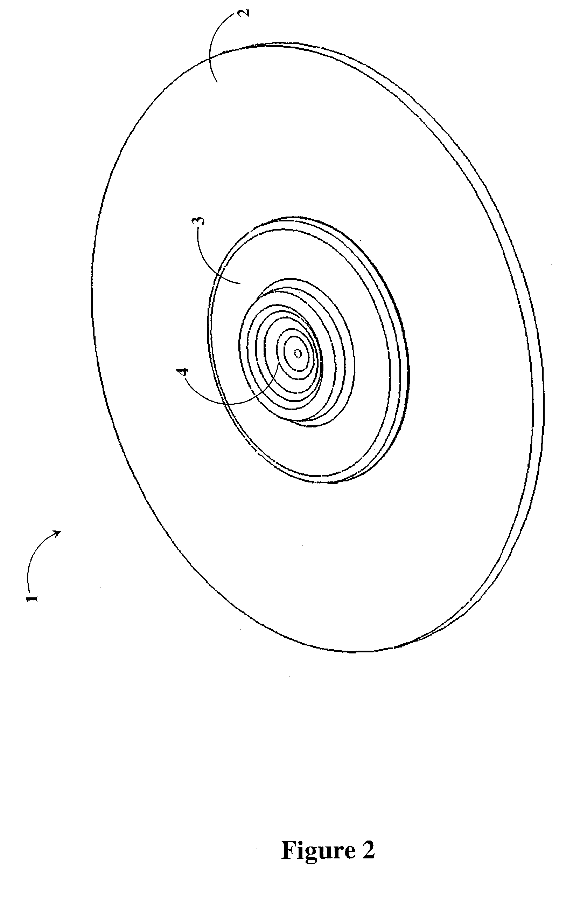

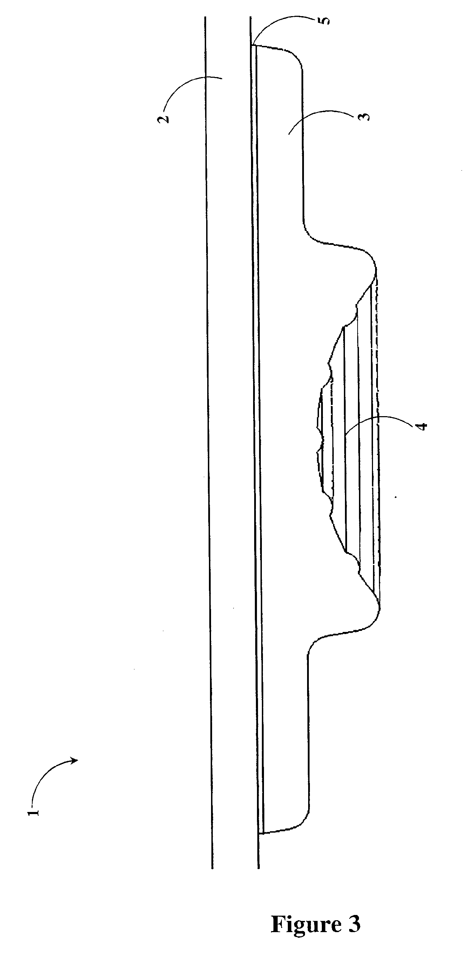

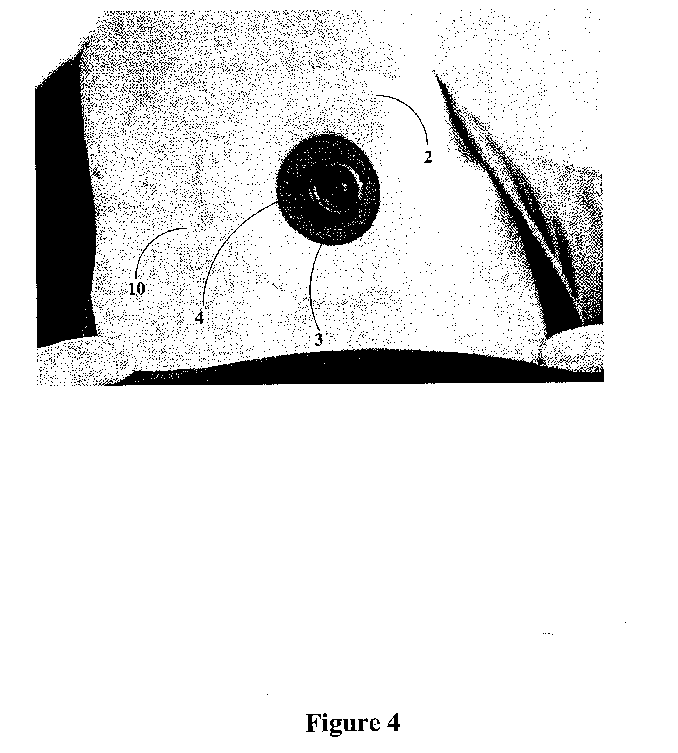

Figure 2 shows a perspective view of the embodiment shown in Figure 1, Figure 3 shows the advantageous flattened profile of the device as hereinbefore discussed and Figure 4 shows a photographic image of the embodiment shown in Figure 1 *in situ* on a model patient 10.

In an alternative embodiment to that described in Figures 1 to 4 an integral tracheostomy dressing is provided wherein the adhesive layer 5 is omitted and the wound locating portion 3 is moulded integrally with the dressing portion 2.

In use, the tracheostomy dressing is applied to the wound of a patient following decanulation. During use, the patient readily feels the recessed portion 4 which is easily located in an integral position within the wound dressing. This enables firm pressure to be applied in the correct position (that is, directly over the wound) during times when air could preferentially pass via the healing wound rather than the larynx. This occurs during high-pressure expiration such as talking, coughing or sneezing. The pressure on the recessed portion 4 and the ribs means that the finger tip of the patient is less likely to slip off the wound locating portion 3 once it is located.

It is expected that the tracheostomy dressing will be replaced everyday for 1-2 days following decanulation and every 2-3 days thereafter. Thus, it is expected that each patient will typically require approximately 10 dressings.

## Claims

1. A tracheostomy dressing which comprises an integral wound locating device having a textured portion that is (i) recessed or concave, and/or (ii) ribbed, wherein the integral wound locating device is adapted, in use, to allow for the location of a healing tracheostomy wound and the application of pressure thereto.

2. The tracheostomy dressing as defined in claim 1, wherein the tracheostomy dressing comprises a hydrocolloid material.

3. The tracheostomy dressing as defined in claim 1 or claim 2, wherein the tracheostomy dressing is impregnated with one or more antiseptic chemicals.

4. The tracheostomy dressing as defined in any preceding claim, wherein the textured portion has a surface feel that is distinct from the surrounding area.

5. The tracheostomy dressing as defined in any preceding claim, wherein the textured portion is recessed.

6. The tracheostomy dressing as defined in any one of claims 1-4, wherein the textured portion is concave.

7. The tracheostomy dressing as defined in any one of claims 1-4, wherein the textured portion is ribbed.

8. The tracheostomy dressing as defined in any one of claims 1-4, wherein the textured portion is recessed and ribbed.

9. The tracheostomy dressing as defined in any one of claims 1-4, wherein the textured portion is concave and ribbed.

10. The tracheostomy dressing as defined in any preceding claim, wherein the textured portion is substantially square, rectangular or circular in shape.

11. The tracheostomy dressing as defined in claim 10, wherein the textured portion is substantially circular in shape.

12. The tracheostomy dressing as defined in any preceding claim, wherein the wound locating device is constructed from a flexible material.

13. The tracheostomy dressing as defined in claim 12, wherein the wound locating device is constructed from the group consisting of: polyurethane, polyethylene, thermoplastic elastomeric (TPE) material, thermoplastic rubber (TPR) material, a silicone material or a styrene ethylene butylene styrene (SEBS) material.

14. The tracheostomy dressing as defined in any preceding claim, wherein the wound locating device is transparent.

## Patentansprüche

1. Tracheostomieverband, der eine integrale Wundlokalisierungsvorrichtung umfasst, die einen strukturierten Teil aufweist, der (i) vertieft oder konkav, und/oder (ii) gerippt ist, wobei die integrale Wundlokalisierungsvorrichtung dafür ausgelegt ist, bei Gebrauch die Lokalisierung einer heilenden Tracheostomiewunde und die Anwendung von Druck auf dieselbe zu ermöglichen.

2. Tracheostomieverband nach Anspruch 1, wobei der Tracheostomieverband ein Hydrokolloid-Material umfasst.

3. Tracheostomieverband nach Anspruch 1 oder Anspruch 2, wobei der Tracheostomieverband mit einer oder mehreren antiseptischen Chemikalien imprägniert ist.

4. Tracheostomieverband nach einem der vorhergehenden Ansprüche, wobei der strukturierte Abschnitt eine Haptik aufweist, die sich von dem umgebenden Bereich unterscheidet.

5. Tracheostomieverband nach einem der vorhergehenden Ansprüche, wobei der strukturierte Abschnitt vertieft angeordnet ist.

6. Tracheostomieverband nach einem der Ansprüche 1 bis 4, wobei der strukturierte Abschnitt konkav ist.

7. Tracheostomieverband nach einem der Ansprüche 1 bis 4, wobei der strukturierte Abschnitt gerippt ist.

8. Tracheostomieverband nach einem der Ansprüche 1 bis 4, wobei der strukturierte Abschnitt vertieft angeordnet und gerippt ist.

9. Tracheostomieverband nach einem der Ansprüche 1 bis 4, wobei der strukturierte Abschnitt konkav und gerippt ist.

10. Tracheostomieverband nach einem der vorhergehenden Ansprüche, wobei der strukturierte Abschnitt eine im Wesentlichen quadratische, rechteckige oder kreisförmige Form aufweist.

11. Tracheostomieverband nach Anspruch 10, wobei der strukturierte Abschnitt eine im Wesentlichen kreisförmige Form aufweist.

12. Tracheostomieverband nach einem der vorhergehenden Ansprüche, wobei die Wundlokalisierungsvorrichtung aus einem flexiblen Material hergestellt ist.

13. Tracheostomieverband nach Anspruch 12, wobei die Wundlokalisierungsvorrichtung aus einem Material hergestellt ist, das aus der Gruppe ausgewählt wird, die aus Folgendem besteht: Polyurethan, Polyethylen, thermoplastischem elastomerem (TPE) -Material, thermoplastischem Kautschuk (TPR) -Material, einem Silikon-Material oder einem Styrol-Ethylen-Butylen-Styrol (SEBS) -Material.

14. Tracheostomieverband nach einem der vorhergehenden Ansprüche, wobei die Wundlokalisierungsvorrichtung transparent ist.

## Revendications

1. Pansement de trachéostomie qui comporte un dispositif intégral de localisation de plaie ayant une partie texturée qui est (i) en retrait ou concave, et/ou (ii) nervurée, dans lequel le dispositif intégral de localisation de plaie est adapté, lors de l'utilisation, pour permettre la localisation d'une plaie de trachéostomie cicatrisante et l'application de pression sur celle-ci.

2. Pansement de trachéostomie selon la revendication 1, dans lequel le pansement de trachéostomie comporte un matériau à l'hydrocolloïde.

3. Pansement de trachéostomie selon la revendication 1 ou la revendication 2, dans lequel le pansement de trachéostomie est imprégné d'un ou de plusieurs produits chimiques antiseptiques.

4. Pansement de trachéostomie selon l'une quelconque des revendications précédentes, dans lequel la partie texturée a un toucher de surface qui est distinct de la zone environnante.

5. Pansement de trachéostomie selon l'une quelconque des revendications précédentes, dans lequel la partie texturée est en retrait.

6. Pansement de trachéostomie selon l'une quelconque des revendications 1 à 4, dans lequel la partie texturée est concave.

7. Pansement de trachéostomie selon l'une quelconque des revendications 1 à 4, dans lequel la partie texturée est nervurée.

8. Pansement de trachéostomie selon l'une quelconque des revendications 1 à 4, dans lequel la partie texturée est en retrait et nervurée.

9. Pansement de trachéostomie selon l'une quelconque des revendications 1 à 4, dans lequel la partie texturée est concave et nervurée.

10. Pansement de trachéostomie selon l'une quelconque des revendications précédentes, dans lequel la partie texturée est d'une forme sensiblement carrée, rectangulaire ou circulaire.

11. Pansement de trachéostomie selon la revendication 10, dans lequel la partie texturée est d'une forme sensiblement circulaire.

12. Pansement de trachéostomie selon l'une quelconque des revendications précédentes, dans lequel le dispositif de localisation de plaie est construit à partir d'un matériau souple.

13. Pansement de trachéostomie selon la revendication 12, dans lequel le dispositif de localisation de plaie est construit à partir du groupe constitué par : du polyuréthane, du polyéthylène, un matériau élastomère thermoplastique (TPE), un matériau caoutchouc thermoplastique (TPR), un matériau à la silicone ou un matériau styrène-éthylène-butylène-styrène (SEBS).

14. Pansement de trachéostomie selon l'une quelconque des revendications précédentes, dans lequel le dispositif de localisation de plaie est transparent.
